# EUROPEAN PATENT APPLICATION

(11) **EP 4 542 249 A2**
(43) Date of publication of application: **23.04.2025**
(21) Application number: 24206071.3
(22) Date of filing: 11.10.2024
(51) Int. Cl.: G01R 33/54, A61B 5/055, A61B 6/03, G01R 33/56

(54) **A METHOD FOR MODIFYING MEDICAL IMAGING PROTOCOL PARAMETERS**

(30) Priority: 16.10.2023 GB 202315829
(71) Applicant: Elekta Limited, Crawley, Sussex RH10 9RR (GB)
(72) Inventor: FREEDMAN, Joshua, Crawley, RH10 9RR (GB); GROSS, Patrick, Crawley, RH10 9RR (GB); ROBERTS, David, Crawley, RH10 9RR (GB); STEMKENS, Bjorn, Crawley, RH10 9RR (GB)
(74) Representative: Thomas, Tomos Daniel

(57) **Abstract**

Disclosed herein are approaches for modifying medical imaging protocol parameters. A computer implemented method for modifying medical imaging protocol parameters comprises receiving modified protocol parameters for an imaging protocol; and determining whether the modified protocol parameters are acceptable based on at least one of an acquired image acceptability check and a parameter acceptability check.

## Description

This disclosure relates to medical imaging protocols, and in particular to a method and system for modifying medical imaging protocol parameters.

### Technical Field

Radiotherapy can be described as the use of ionising radiation, such as X-rays, to treat a human or animal body. Radiotherapy is commonly used to treat tumours within the body of a patient or subject. In such treatments, ionising radiation is used to irradiate, and thus destroy or damage, cells which form part of the tumour.

A radiotherapy device typically comprises a gantry which supports a beam generation system, or other source of radiation, which is rotatable around a patient. For example, for a linear accelerator (linac) device, the beam generation system may comprise a source of radio frequency energy, a source of electrons, an accelerating waveguide, beam shaping apparatus, etc.

A radiotherapy device may also comprise a medical imaging apparatus, such as a magnetic resonance (MR) imaging apparatus, configured to obtain images of a subject. Images may be taken immediately prior to treatment delivery in order to adapt or recalculate the treatment plan, for example to account for inter-fractional changes in patient anatomy. A limitation of this approach is that artefacts exhibited by the acquired images could affect the quality of the image analysis and hence the quality of the adapted treatment plan. For example, image artefacts could affect the quality of image registration between pre-treatment computed tomography (CT) and daily pre-beam MR images. Alternatively, in automated imaging workflows, image artefacts could detrimentally affect the quality of the image analysis using deep learning techniques. For example, image artefacts could detrimentally affect the quality of contours or synthetic CT calculated from a daily MR image using deep learning techniques. MR or other medical imaging may also be used for motion-management purposes to support gated or tracked deliveries of radiotherapy.

There are numerous sources of MR imaging artefacts which could affect an MR image and hence its impact on a radiotherapy workflow. For example, metallic implants in a patient may result in artefacts, such as signal drop-out and/or geometrical distortions, due to local susceptibility-related magnetic field inhomogeneity. Motion of a patient (for example respiratory motion, cardiac cycle, peristalsis, bulk movement, etc.) can lead to ghosting artefacts. For Cartesian sequences, ghosting artefacts manifest along the phase-encoding direction. Additional artefacts include, but are not limited to, Gibbs ringing, chemical shift, B1 inhomogeneities, radial streaking and other artefacts which are known to the skilled person.

Image artefacts can be minimised by optimising protocol parameters of imaging protocols. Image artefacts may also be reduced by careful sequence choice. For example, geometrical distortions can be reduced by applying a three-dimensional (3D) distortion correction and/or by increasing receiver bandwidth. However, the presence and severity of artefacts is patient-dependent and therefore protocol parameter optimisation may also vary between patients. Known imaging apparatuses may only allow use of particular validated protocols, which are locked and do not allow any protocol parameters to be changed or customised or optimised for different patients and/or clinical workflows.

There is a need to allow users to use, modify, customise and/or optimise imaging protocols, optionally as part of a clinical workflow.

The present invention seeks to address these and other disadvantages encountered in the prior art.

### Summary

An invention is set out in the claims.

### Brief Description of the Drawings

Specific examples are now described, by way of example only, with reference to the drawings, in which:
Figure 1 depicts a radiotherapy device or apparatus according to the present disclosure;
Figure 2 depicts a method for modifying medical imaging protocol parameters according to the present disclosure;
Figure 3 depicts an acquired image acceptability check according to the present disclosure;
Figure 4 depicts a parameter acceptability check according to the present disclosure;
Figure 5 depicts a radiotherapy device comprising a computing system according to the present disclosure;
Figure 6 depicts a computer program product according to the present disclosure.

### Overview

A method for modifying medical imaging protocol parameters is provided, comprising receiving modified protocol parameters for an imaging protocol; and determining whether the modified protocol parameters are acceptable based on at least one of an acquired image acceptability check and a parameter acceptability check. This means that modified protocol parameters can be assessed based on their effect on acquired images, and/or based on the parameters themselves. This provides customisability without compromise to image quality or imaging protocol functionality, and within conservative boundaries to ensure safety and comfort for the patient.

### Detailed Description

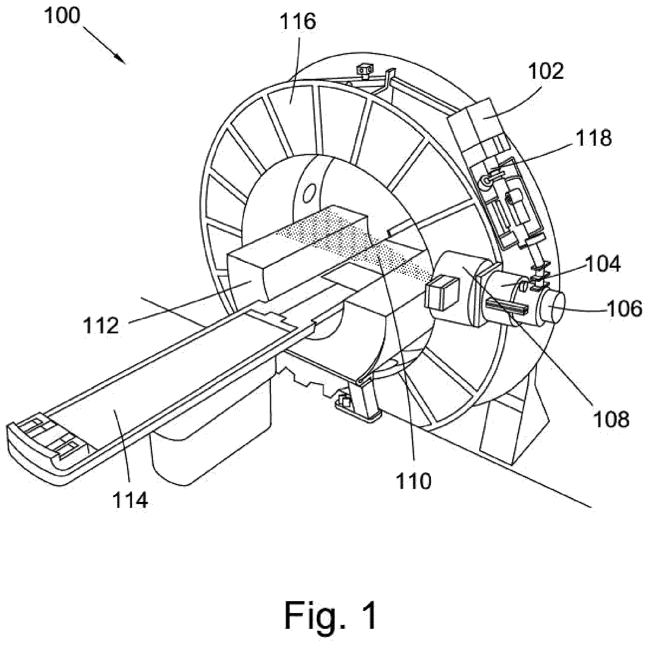

Figure 1 depicts a radiotherapy device suitable for delivering, and configured to deliver, a beam of radiation to a patient during radiotherapy treatment. The device and its constituent components will be described generally for the purpose of providing useful accompanying information for the present invention. The device depicted in Figure 1 is in accordance with the present disclosure and is suitable for use with the disclosed systems and apparatuses. While the device in Figure 1 is an MR-linac, the implementations of the present disclosure may be any radiotherapy device, for example a linac device.

The device 100 depicted in Figure 1 is an MR-linac. The device 100 comprises both MR imaging apparatus 112 and radiotherapy (RT) apparatus which may comprise a linac device. The MR imaging apparatus 112 is shown in cross-section in the diagram. In operation, the MR scanner produces MR images of the patient, and the linac device produces and shapes a beam of radiation and directs it toward a target region within a patient's body in accordance with a radiotherapy treatment plan. The depicted device does not have the usual 'housing' which would cover the MR imaging apparatus 112 and RT apparatus in a commercial setting such as a hospital.

The MR-linac device depicted in Figure 1 comprises a source of radiofrequency waves 102, a waveguide 104, a source of electrons 106, a source of radiation 106, a collimator 108 such as a multileaf collimator configured to collimate and shape the beam, MR imaging apparatus 112, and a patient support surface 114. In use, the device would also comprise a housing (not shown) which, together with the ring-shaped gantry, defines a bore. The moveable support surface 114 can be used to move a patient, or other subject, into the bore when an MR scan and/or when radiotherapy is to commence. The MR imaging apparatus 112, RT apparatus, and a subject support surface actuator are communicatively coupled to a controller or processor. The controller is also communicatively coupled to a memory device comprising computer-executable instructions which may be executed by the controller.

The RT apparatus comprises a source of radiation and a radiation detector (not shown). Typically, the radiation detector is positioned diametrically opposed to the radiation source. The radiation detector is suitable for, and configured to, produce radiation intensity data. In particular, the radiation detector is positioned and configured to detect the intensity of radiation which has passed through the subject. The radiation detector may also be described as radiation detecting means, and may form part of a portal imaging system.

The radiation source may comprise a beam generation system. For a linac, the beam generation system may comprise a source of RF energy 102, an electron gun 106, and a waveguide 104. The radiation source is attached to the rotatable gantry 116 so as to rotate with the gantry 116. In this way, the radiation source is rotatable around the patient so that the treatment beam 110 can be applied from different angles around the gantry 116. In a preferred implementation, the gantry is continuously rotatable. In other words, the gantry can be rotated by 360 degrees around the patient, and in fact can continue to be rotated past 360 degrees. The gantry may be ring-shaped. In other words, the gantry may be a ring-gantry.

The source 102 of radiofrequency waves, such as a magnetron, is configured to produce radiofrequency waves. The source 102 of radiofrequency waves is coupled to the waveguide 104 via circulator 118, and is configured to pulse radiofrequency waves into the waveguide 104. Radiofrequency waves may pass from the source 102 of radiofrequency waves through an RF input window and into an RF input connecting pipe or tube. A source of electrons 106, such as an electron gun, is also coupled to the waveguide 104 and is configured to inject electrons into the waveguide 104. In the electron gun 106, electrons are thermionically emitted from a cathode filament as the filament is heated. The temperature of the filament controls the number of electrons injected. The injection of electrons into the waveguide 104 is synchronised with the pumping of the radiofrequency waves into the waveguide 104. The design and operation of the radiofrequency wave source 102, electron source and the waveguide 104 is such that the radiofrequency waves accelerate the electrons to very high energies as the electrons propagate through the waveguide 104.

The design of the waveguide 104 depends on whether the linac accelerates the electrons using a standing wave or travelling wave, though the waveguide typically comprises a series of cells or cavities, each cavity connected by a hole or 'iris' through which the electron beam may pass. The cavities are coupled in order that a suitable electric field pattern is produced which accelerates electrons propagating through the waveguide 104. As the electrons are accelerated in the waveguide 104, the electron beam path is controlled by a suitable arrangement of steering magnets, or steering coils, which surround the waveguide 104. The arrangement of steering magnets may comprise, for example, two sets of quadrupole magnets.

Once the electrons have been accelerated, they may pass into a flight tube. The flight tube may be connected to the waveguide by a connecting tube. This connecting tube or connecting structure may be called a drift tube. The electrons travel toward a heavy metal target which may comprise, for example, tungsten. Whilst the electrons travel through the flight tube, an arrangement of focusing magnets act to direct and focus the beam on the target.

To ensure that propagation of the electrons is not impeded as the electron beam travels toward the target, the waveguide 104 is evacuated using a vacuum system comprising a vacuum pump or an arrangement of vacuum pumps. The pump system is capable of producing ultra-high vacuum (UHV) conditions in the waveguide 104 and in the flight tube. The vacuum system also ensures UHV conditions in the electron gun. Electrons can be accelerated to speeds approaching the speed of light in the evacuated waveguide 104.

The source of radiation is configured to direct a beam 110 of therapeutic radiation toward a patient positioned on the patient support surface 114. The source of radiation may comprise a heavy metal target toward which the high energy electrons exiting the waveguide are directed. When the electrons strike the target, X-rays are produced in a variety of directions. A primary collimator may block X-rays travelling in certain directions and pass only forward travelling X-rays to produce a treatment beam 110. The X-rays may be filtered and may pass through one or more ion chambers for dose measuring. The beam can be shaped in various ways by beam-shaping apparatus, for example by using a multileaf collimator 108, before it passes into the patient as part of radiotherapy treatment.

In some implementations, the source of radiation is configured to emit either an X-ray beam or an electron particle beam. Such implementations allow the device to provide electron beam therapy, i.e. a type of external beam therapy where electrons, rather than X-rays, are directed toward the target region. It is possible to 'swap' between a first mode in which X-rays are emitted and a second mode in which electrons are emitted by adjusting the components of the linac. In essence, it is possible to swap between the first and second mode by moving the heavy metal target in or out of the electron beam path and replacing it with a so-called 'electron window'. The electron window is substantially transparent to electrons and allows electrons to exit the flight tube.

The subject or patient support surface 114 is configured to move between a first position substantially outside the bore, and a second position substantially inside the bore. In the first position, a patient or subject can mount the patient support surface. The support surface 114, and patient, can then be moved inside the bore, to the second position, in order for the patient to be imaged by the M R imaging apparatus 112 and/or imaged or treated using the RT apparatus. The movement of the patient support surface is effected and controlled by a subject support surface actuator, which may be described as an actuation mechanism. The actuation mechanism is configured to move the subject support surface in a direction parallel to, and defined by, the central axis of the bore. The terms subject and patient are used interchangeably herein such that the subject support surface can also be described as a patient support surface. The subject support surface may also be referred to as a moveable or adjustable couch or table.

The radiotherapy apparatus / device depicted in Figure 1 also comprises MR imaging apparatus 112. The MR imaging apparatus 112 is configured to obtain or acquire images of a subject positioned, i.e. located, on the subject support surface 114. The MR imaging apparatus 112 may also be referred to as the MR imager. The MR imaging apparatus 112 may be a conventional MR imaging apparatus operating in a known manner to obtain or acquire MR data, for example MR images. The skilled person will appreciate that such a MR imaging apparatus 112 may comprise a primary magnet, one or more gradient coils, one or more receive coils, and an RF pulse applicator. The operation of the MR imaging apparatus is controlled by the controller.

The controller is a computer, processor, or other processing apparatus. The controller may be formed by several discrete processors; for example, the controller may comprise an MR imaging apparatus processor, which controls the MR imaging apparatus 112; an RT apparatus processor, which controls the operation of the RT apparatus; and a subject support surface processor which controls the operation and actuation of the subject support surface. The controller is communicatively coupled to a memory, e.g. a computer readable medium.

The linac device also comprises several other components and systems as will be understood by the skilled person. For example, in order to ensure the linac does not leak radiation, appropriate shielding is also provided.

The disclosed approach allows for modification of imaging protocol parameters, enabling customisation of imaging protocols whilst ensuring maintained image quality and optimal performance. The imaging protocol of the approaches disclosed herein may relate to MR imaging, or may be an MR imaging protocol, and MR imaging will be used as a particular example to illustrate the disclosure. However, the disclosed approaches may additionally or alternatively relate to another type of medical imaging and medical imaging protocol, such as CT imaging, cone-beam CT imaging, single-photon emission computed tomography (SPECT) imaging, and/or positron emission tomography (PET) imaging.

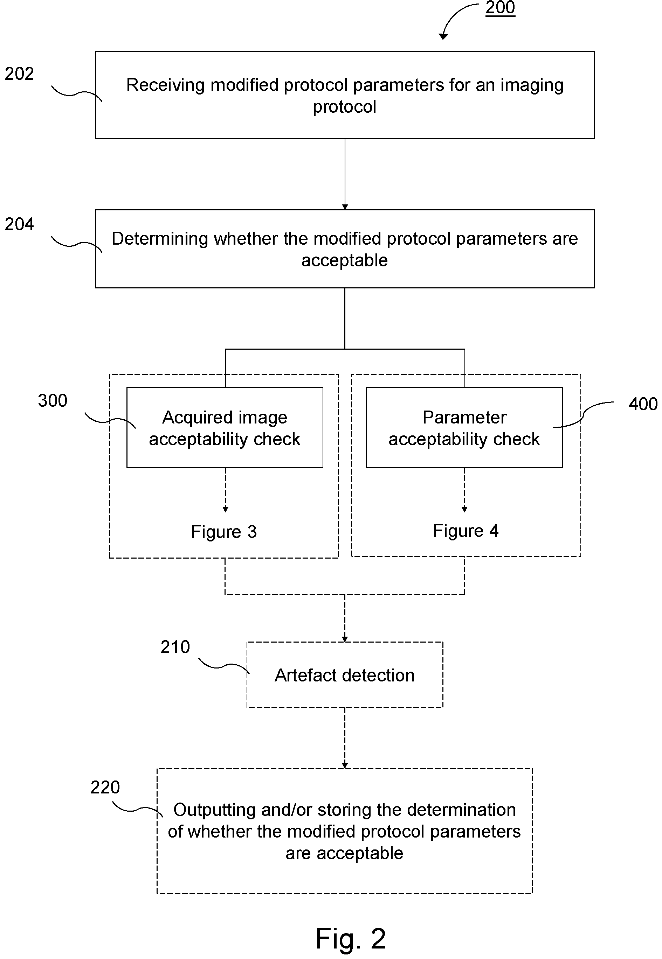

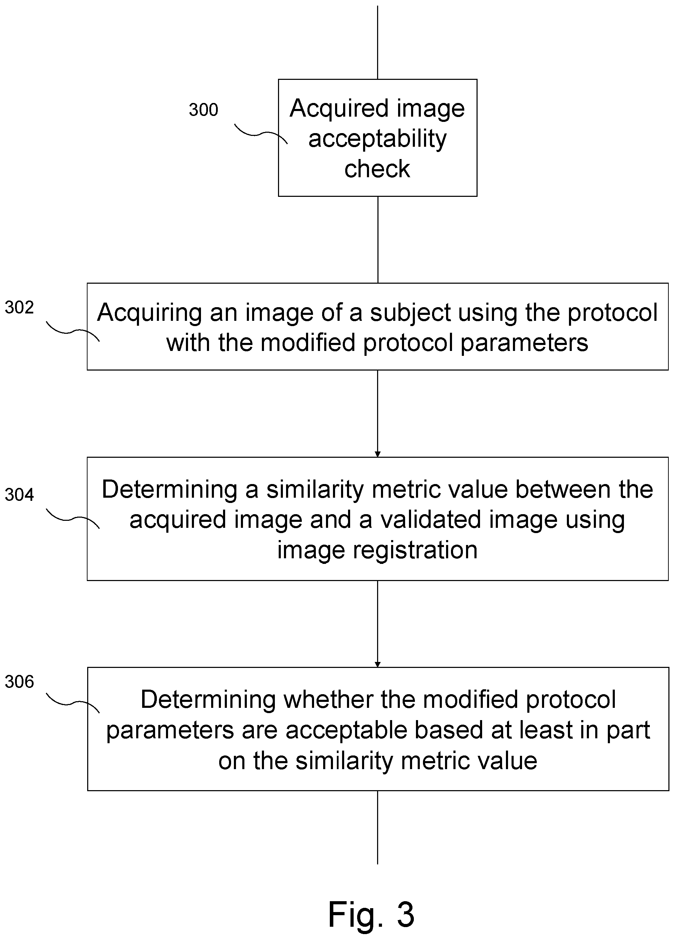

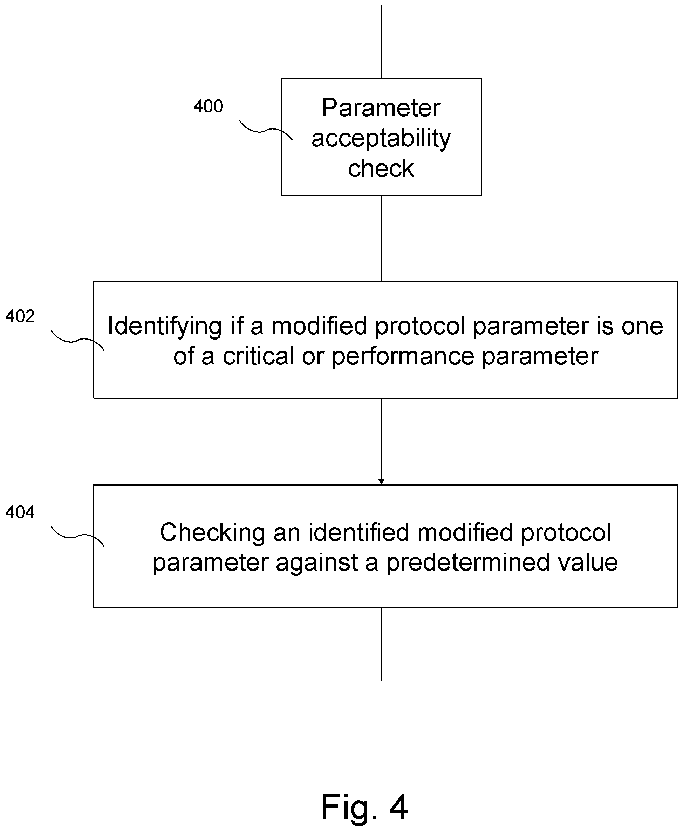

Figure 2 depicts a method 200 for modifying imaging protocol parameters according to an aspect of the present disclosure. The method 200 comprises receiving 202 modified protocol parameters for an imaging protocol; and determining 204 whether the modified protocol parameters are acceptable. The determining 204 whether the modified protocol parameters are acceptable is based on at least one of an acquired image acceptability check 300 and a parameter acceptability check 400. In other words, the present disclosure provides an aspect of a method 200 for modifying imaging protocol parameters relating to assessing acquired image acceptability, and an aspect of a method 200 for modifying medical imaging protocol parameters relating to assessing parameter acceptability. An acquired image acceptability check according to the present disclosure is depicted in Figure 3, such that Figures 2 and 3 together depict an aspect of the method 200 relating to assessing parameter acceptability. Similarly, Figure 4 depicts a parameter acceptability check according to the present disclosure such that Figures 2 and 4 together depict an aspect of the method 200 relating to assessing parameter acceptability. In some examples of the method 200, both the acquired image acceptability check and parameter acceptability check may be performed.

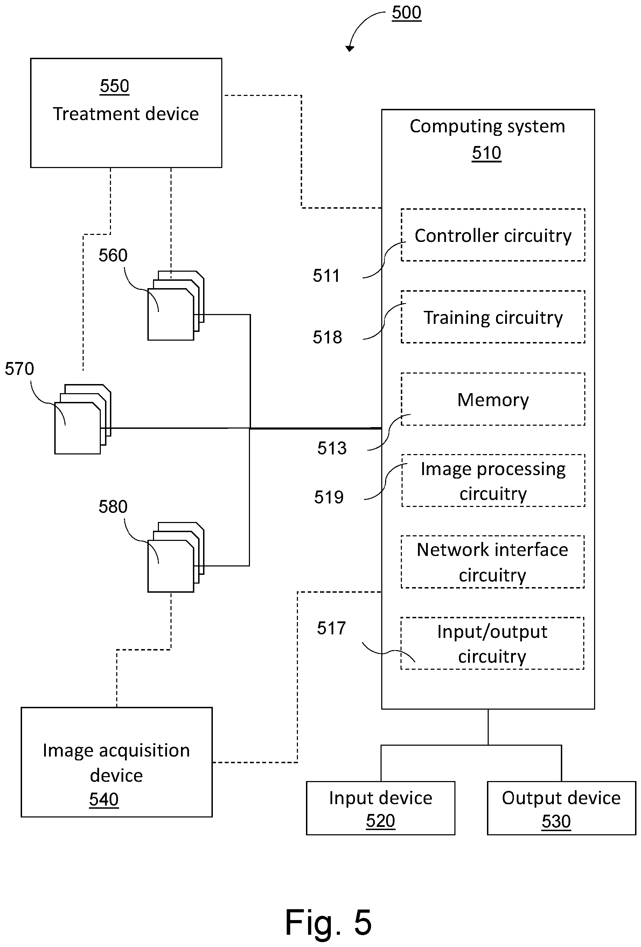

In some examples, the method 200 further comprises performing artefact detection on an acquired image. The method may also comprise outputting and/or storing 220 the determination of whether the modified protocol parameters are acceptable, for determinations performed based on either or both of the acquired image acceptability check and the parameter acceptability check. The method 200 is a computer-implemented method, and may be used in conjunction with, optionally performed by, a radiotherapy device and/or imaging apparatus such as the device 100 of Fig. 1, the system 500 of Fig. 5, the imaging apparatus 112 of Fig. 1, and/or the image acquisition device 540 of Fig. 5.

The method 200 comprises receiving 202 modified protocol parameters for an imaging protocol. The receiving 202 modified protocol parameters for an imaging protocol may comprise obtaining, inputting, or accessing the modified protocol parameters for an imaging protocol, for example by or at an imaging apparatus. In some examples, the modified protocol parameters are input by a user. The method 200 may further comprise prompting a user to input the modified protocol parameters. The parameters may be input at an input device such as a user input device. The input device may comprise a user interface, optionally a graphical user interface (GUI), and may be located at or comprised in a radiotherapy device 100 and/or an imaging apparatus 112 and/or a computing device communicatively coupled to the radiotherapy device and/or imaging apparatus. In some examples, a user inputs modified protocol parameters through any means disclosed herein, and the modified protocol parameters are transmitted and/or received, for example transmitted from the input device and/or received by the imaging apparatus 112, for use in the present method. Receiving the modified protocol parameters from a user input device allows intuitive interaction between a user and the present method and systems, providing easy-to-operate means of customising medical imaging protocols. The modified protocol parameters may also be received from a database, and/or be based at least in part on a treatment plan for a subject. The term 'subject' as used herein may refer to a patient or a particular section or subsection of a patient.

The present method comprising receiving 202 modified protocol parameters for an imaging protocol allows customisation of an imaging protocol, enabling modification of protocol parameters to adjust the protocol for a given patient and/or workflow. This allows artefacts to be reduced or prevented, improving image quality and improving the quality of adapted or recalculated treatment plans using the images. Furthermore, the present method facilitates the modification of protocol parameters through an authorised or permitted process. This reduces the risk of users attempting to edit or modify protocols via unauthorised or unsupervised means or improvised workarounds.

The method 200 comprises determining 204 whether the modified protocol parameters are acceptable. The determining is based on at least one of an acquired image acceptability check 300 and a parameter acceptability check 400. In some examples, the method 200 further comprises performing at least one of the acquired image acceptability check and the parameter acceptability check. Examples of the acquired image acceptability check 300 and parameter acceptability check 400 will be described in more detail with respect to Figures 3 and 4, respectively.

Figure 3 depicts an acquired image acceptability check according to an aspect of the method 200 for modifying medical imaging protocol parameters. In this aspect, the method 200 comprises receiving 202 modified protocol parameters for an imaging protocol; and determining 204 whether the modified protocol parameters are acceptable based on at least an acquired image acceptability check 300. The acquired image acceptability check 300 comprises acquiring 302 an image of a subject using the protocol with the modified protocol parameters; determining 304 a similarity metric value between the acquired image and a validated image using image registration; and determining 306 whether the modified protocol parameters are acceptable based at least in part on the similarity metric value.

The acquiring 302 an image of a subject using the protocol with the modified parameters comprises obtaining image data. In examples in which the imaging protocol relates to MR imaging, the image acquisition and/or obtaining of image data is performed by the MR imaging apparatus 112. An imaging protocol may comprise a plurality of protocol parameters. At least one of the plurality of protocol parameters of an imaging protocol may be modified. The received modified protocol parameters correspond to some or all of the plurality of protocol parameters of a given imaging protocol. In other words, the imaging protocol with the modified protocol parameters may also comprise one or more other protocol parameters which are not modified. In some examples, protocol parameters which are not modified may include at least one of an MR sequence type parameter and/or which RF coils are to be used for imaging. In some examples, an MR sequence type may be selected and then at least one parameter may be subsequently modified (such as a field of view parameter), while other parameters are left unchanged (such as a TR/TE parameter). Such modifications may be different for different patients, e.g. for one patient a TR/TE parameter may be modified and a field of view parameter is left unchanged, and for another patient a TR/TE parameter may be unchanged and a field of view parameter is modified. As another example, the imaging protocol may comprise a fat suppression parameter value that has been modified from a previous fat suppression parameter value and the imaging protocol may also comprise a specific absorption rate (SAR) parameter that has not been modified away from the SAR parameter that was to be used with the previous fat suppression parameter value. Further examples of parameters that may be left unmodified include at least one of: the type of RF pulse that is used, the maximum strength of the gradients/slope, and/or the coil that is used.

In some examples, images are acquired using the imaging protocol with unmodified or validated protocol parameters. Protocol parameters which are not modified may be received as part of method 200. Protocol parameters which are not modified take a 'default' or 'standard' or pre-set value and/or may be automatically determined based at least in part on the modified parameters. Default or standard or pre-set protocol parameters are set as part of, and may vary between, given imaging protocols. An imaging protocol corresponds to a validated image, such that the validated image is acquired using the imaging protocol with no modified protocol parameters, and the acquired image of the present method is acquired using the modified protocol parameters. In this way, a user can start from an imaging protocol and corresponding validated image, modify the protocol by providing modified protocol parameters and then acquire an image using the imaging protocol with the modified parameters. The acquired image is then used in a registration step. The registration step comprises registering, or attempting to register, the acquired image to the validated image, and may use conventional image registration techniques that will be known to those skilled in the art. The registration step can be used to determine whether the modified protocol parameters are acceptable based on how, or if, the acquired image differs from the validated image. The registration step may include an alignment step that uses mutual information from the acquired image and validated image. The registration step may include using a viewer to switch between the two images to evaluate how similar the images are and whether they are registered correctly.

Various alternative image registration techniques are possible, which may each comprise the following exemplary steps:
1) Determining the fixed/target image and the moving/source image. The moving image is registered to the fixed image.
2) Determining registration type, parameters and domain. There are different types of registration (linear, rigid, non-rigid, etc.) parameters. Commonly, the domain in which the registration is done is the image domain.
3) Ensuring the resolution is the same for both acquisitions, such that pixels/voxels can be compared to each other.
4) Performing registration by looking at corresponding features in both images, or looking at intensities that are similar by calculation of correlation metrics between the images (e.g. corresponding lines, contours, etc.). This is followed by attempting to maximise the correspondence by changing the moving/source image. Mathematically this is done by minimizing a cost function. The higher the correspondence between both images, the lower the cost function, and so the images may be considered registered when the cost-function is minimised.

Further steps may be taken to improve image registration such as, for example, by performing the registration at different resolution levels (first coarse, and then fine resolution), or by using prior information about the underlying anatomy (e.g. brains will not deform, but they will rotate).

The present method comprising acquiring 302 an image of a subject using the protocol with modified parameters facilitates a comparison between the acquired image acquired with the modified protocol parameters, and the validated image. This allows modified protocol parameters and the image acquired to be tested against, or compared to or evaluated based on, validated images and/or validated protocols. Accordingly, protocol parameters can be modified without compromising imaging functionality, quality or performance. In examples in which the imaging protocol relates to MR imaging or is an MR imaging protocol, the acquired and validated images may each be MR images.

In one approach, the similarity metric value is associated with a geometrical transformation between the acquired image and a validated image. Determining 304 a similarity metric value between the acquired image and a validated image using image registration comprises determining a geometrical transformation between the acquired image and the validated image. The geometrical transformation between the acquired image and the validated image may be determined by, for example, using an approach based on comparing respective pixel values of the two images. As explained with reference to step 302, the validated image may be an image acquired using the imaging protocol with no modified protocol parameters, i.e. using a 'standard' or 'pre-set' or 'validated' imaging protocol. The geometrical transformation serves as a measure of similarity or geometrical fidelity between the acquired image and the validated image. For example, a minimal transformation suggests that the acquired image is geometrically similar to the validated image, and as such that the modified parameters do not result in any unacceptable changes to image acquisition or the acquired image. The determining 306 whether the modified protocol parameters are acceptable is based at least in part on the geometrical transformation. Whilst the modified parameters may reduce or prevent image artefacts, the overall structures or features of the image may remain substantially the same or similar. In other words, a reduction or prevention of image artefacts is needed, however, minimal deviation from a validated image with respect to other aspects may be desirable. Therefore, with the present method for modifying imaging protocol parameters, image quality can be improved with respect to reducing artefacts, and other reductions in image quality or otherwise detrimental effects to the image acquisition or acquired image can be monitored and/or avoided.

In some examples, the geometrical transformation comprises a deformation vector field. The deformation vector field may comprise x, y and/or z displacement components. That is, the deformation vector field may comprise at least one x displacement component, at least one y displacement component and/or at least one z displacement component. The deformation vector field may comprise a plurality of x displacement components, a plurality of y components and/or a plurality of z components. One or more of the displacement components comprises a scalar length. Determining the geometrical transformation comprises determining at least one of the deformation vector field, at least one of an x, y and/or z displacement component of the deformation vector field and/or a scalar length of at least one of the displacement components. The determining 306 whether the modified protocol parameters are acceptable is based at least in part on at least one of an x, y and/or z displacement component of the deformation vector field and/or a scalar length of at least one of the displacement components.

A registration step may comprise attempting to register, and/or successfully registering the acquired image to a validated image. If an attempt to register the acquired image to a validated image is unsuccessful, i.e. if the acquired image cannot be registered to the validated image, or a geometrical transformation between the acquired image and a validated image cannot be determined, the modified protocol parameters may be determined to be unacceptable. This ensures that modified protocol parameters can be identified which result in a disadvantageous change to image quality. The modified protocol parameters and/or their determination that they are unacceptable may be output and/or stored 220, to improve the efficiency of the method if the same modified protocol parameters are received in the future, and to allow review of unacceptable modified protocol parameters independent from performance of the method 200. In some examples, if the modified protocol parameters are determined to be acceptable, protocol parameters are updated based on the modified protocol parameters. The protocol parameters to be updated may be, for example, stored protocol parameters, validated protocol parameters, and/or original protocol parameters.

In some aspects of the present disclosure relating to assessing acquired image acceptability, the similarity metric value is associated with a structural similarity index metric, or a normalized cross correlation metric, between the acquired image and the validated image. Techniques for determining a structural similarity index metric or normalized cross correlation metric will be known to those skilled in the art. A structural similarity index approach may identify at least one structure (such as a circle) in one of the images and attempt to identify that structure, or a similar structure, in the other image. A structural similarity index approach may involve analysis of luminance, contrast, and structure within the image and calculation of a single value per pixel. Structural similarity index metrics give a relative comparison and error value, rather than an absolute error value. A normalized cross correlation approach may comprise comparing pixel values and determining if a pixel is at a minimum, such as by working out the distance to a similar pixel value. A normalized cross correlation approach may comprise normalising the data prior to calculating the difference between the pixels in both images. A normalized cross correlation metric provides a relative comparison between the two images.

The present method comprising determining 304 a similarity metric value between the acquired image and a validated image using image registration facilitates effective comparison between validated images and images acquired using an imaging protocol with modified protocol parameters. This means modified protocol parameters can be evaluated, assessing any changes to the acquired image compared to the validated image.

An aspect of the method 200 relating to assessing acquired image acceptability comprises determining 306 whether the modified protocol parameters are acceptable based at least in part on the similarity metric. In general, the determining whether the modified protocol parameters are acceptable based at least in part on the similarity metric may comprise determining whether the similarity metric is an acceptable metric. In some examples, this comprises determining whether the deformation vector field of a geometrical transformation comprises displacement components on an acceptable scale. In other words, modified protocol parameters may be determined to be acceptable if a geometrical transformation, or other similarity metric, between the acquired image and the validated image corresponds to a sufficiently small or acceptable difference between the acquired image and the validated image.

In some examples, whether the modified protocol parameters are acceptable is determined based at least in part on a deformation vector field of the geometrical transformation, at least one of an x, y and/or z displacement component of the deformation vector field, and/or on a scalar length of at least one of the displacement components.

In an aspect of the method 200 relating to assessing acquired image acceptability, modified protocol parameters are determined to be acceptable if the acquired image is within a certain transformation of the validated image. In particular, modified protocol parameters are determined to be acceptable if a certain proportion of the x, y and/or z components of the deformation vector field have a scalar length less than or equal to, or strictly less than, a maximum scalar length. The certain proportion may be referred to as a threshold proportion, an optimal proportion, a minimum proportion or a reference proportion. The maximum scalar length may be referred to as a threshold scalar length or a reference scalar length. Accordingly, the modified protocol parameters are determined to be acceptable if a scalar length of a threshold proportion of the x, y and/or z displacement components are less than or equal to a maximum scalar length. In some examples, the maximum scalar length is a value within the range of no less than 0.5 mm to no greater than 4mm. In other examples, the maximum scalar length is a value within the range of no less than 1.5 mm to no greater than 2.5mm, most preferably around 2 mm. The threshold proportion may be a value within a range from no less than 60% to no greater than 100%, or within a range from no less than 80% to no greater than 100%, or within a range from no less than 90% to no greater than 100%, and most preferably may be 95%.

In some examples, determining 306 that the modified protocol parameters are acceptable comprises determining that at least one of the x, y, and/or z displacement components and/or a scalar length of at least one of the displacement components is less than or equal to, or strictly less than, a distance corresponding to the resolution of the acquired and/or validated image. In other words, the determining 306 comprises determining whether the deformation vector field of a geometrical transformation comprises displacement components on a scale corresponding to the image resolution of the acquired and/or validated image, and/or that the maximum scalar length corresponds to the image resolution, or to a scale or distance corresponding to the image resolution. The maximum scalar length may thus be based on a resolution of the validated image. If the displacement component(s) and/or scalar length of the displacement component(s) are less than or equal to, or strictly less than, the distance or scale corresponding to the image resolution, the modified protocol parameters are determined to be acceptable. If the displacement component(s) and/or scalar length of the displacement component(s) are greater than (or, in some examples, equal to) the distance or scale corresponding to the image resolution, the modified protocol parameters are determined to be not acceptable.

In some examples, the distance or scale corresponding to the resolution of the images is around 1 mm. Accordingly, in some examples, if the maximum scalar length is less than or equal to, or strictly less than, 1 mm, the modified protocol parameters are determined to be acceptable. In some examples, each of the components of the deformation vector field must be less than or equal to the distance corresponding to the resolution of the images for the modified protocol parameters to be determined to be acceptable. In some examples, a threshold proportion of the scalar length of the x, y and/or z components of the deformation vector field must be less than or equal to the distance corresponding to the resolution of the images for the modified protocol parameters to be determined to be acceptable.

Modified imaging protocols with acceptable modified protocol parameters may be considered to be geometrically accurate.

In some examples, it is not necessary to assess the entire acquired image, or every pixel of the acquired image, for the acquired image acceptability check 300. Instead, a portion of the image, or a portion of the pixels of the image, may be assessed for the acquired image acceptability check 300 using the approaches described herein, along with a corresponding portion of the validated image. The portion may be defined by a radius. The portion may correspond to a sub-image of the target of the radiotherapy. The portion may correspond to a sub-image of the target and body contour information of the patient. Likewise, the threshold used by the acceptability check 300 may be determined by the intended purpose of the imaging, which may be only for visualising the tumour, or may also require good visualisation of the body contour(s), such as if the image is to be used for plan calculation.

The determination 306 of whether the modified protocol parameters are acceptable may be output, and/or stored 220. The modified protocol parameters may also be stored along with their respective determination of acceptability to avoid the need to repeat the method 200 if identical modified protocol parameters are received in the future. This may increase the efficiency of the present method, reduce the computational resources required to perform the method and may allow review of stored modified protocol parameters and their acceptability or not independent of performance of the method 200.

Aspects of the present method comprising determining 306 whether the modified protocol parameters are acceptable based at least in part on a similarity metric facilitates evaluation of modified protocol parameters to ensure they do not detrimentally affect image quality or image acquisition functionality. Since the similarity metric between the acquired image and the validated image indicates a degree of fidelity between the acquired image and the validated image, modified protocol parameters can be identified as acceptable or not depending on the degree of variation between the acquired image and the validated image. Therefore, the present method can ensure modified protocol parameters are only accepted where they do not result in disadvantageous changes to the image or other unacceptable or suboptimal effects to image quality. Accordingly, the present method of modifying medical imaging protocol parameters allows customisation, modification and optimisation of imaging protocols whilst mitigating against detrimental effects to image quality or protocol performance which may result from unacceptable modified protocol parameters.

Additional or alternative measures may be implemented to verify or evaluate modified protocols, for example comprising a parameter acceptability check. Figure 4 depicts a parameter acceptability check according to an aspect of the method 200 for modifying medical imaging protocol parameters. In this aspect, the method 200 comprises receiving 202 modified protocol parameters for an imaging protocol; and determining 204 whether the modified protocol parameters are acceptable based on at least a parameter acceptability check 400. The parameter acceptability check 400 comprises identifying 402 if a modified protocol parameter is one of a critical or performance parameter, and checking 404 an identified modified protocol parameter against a predetermined value.

A protocol may only be modifiable away from a validated protocol to a certain degree, e.g. by only allowing modification of a particular set of protocol parameters, or only allowing modification of protocol parameters within a specific range. This enables modification, customisation and optimisation of an imaging protocol whilst limiting the scope of modification, for example to avoid deviation from optimal performance or to ensure modified protocol parameters are compatible with the imaging protocol as a whole, in particular any parameters which are not modified.

In one aspect, the parameter acceptability check comprises identifying 402 if a modified protocol parameter is one of a critical or performance parameter. Acceptable modifications to protocol parameters may depend on whether a modified protocol parameter is one of a critical or performance parameter. A critical parameter is a parameter that corresponds to, for example, at least one of: bandwidth, 3D geometrical distortion, field of view, sound pressure level and specific absorption rate (SAR). A performance parameter is a parameter that corresponds to, for example, at least one of : signal-to-noise ratio (SNR), contrast, view of the body, time of the acquisition, resolution of the acquisition, and fat suppression. The bandwidth parameter may affect the geometric fidelity of the acquired image. SAR gives an indication of heating of the patient (which should be limited). A critical parameter may also correspond to at least one of: parameters related to rotation of the imaging stack; parameters related to use of acceleration techniques, such as sensitivity encoding (SENSE), generalized auto-calibrating partial parallel acquisition (GRAPPA), or compressed SENSE, in particular with high values; and/or a parameters related to a read-out type being Cartesian or non-Cartesian.

In some examples, a given critical or performance parameter corresponds to a predetermined value, or range of values, with respect to which the acceptability of the modified protocol parameter is assessed. Modified protocol parameters identified as one of a critical or performance parameter are checked against a corresponding predetermined value in determining whether the modified protocol parameters are acceptable. The predetermined value depends on factors including the identified modified protocol parameter, other modified protocol parameters, the imaging protocol and/or protocol parameters which are not modified. For example, the performance parameter SNR may be checked against a predetermined value of 10%, such that the SNR of the image acquired with the modified protocol is required to have an SNR parameter of less than or equal to 10% in order that the modified protocol is deemed acceptable. The predetermined value may correspond to critical or performance parameter values of a validated image. A SNR may be kept within 10% of, i.e. differing by less than or equal to 10% of, the value for a validated image. In an exemplar performance of the method 200, a modified SNR is identified as a performance parameter, in particular a modified SNR parameter, and checked against the SNR value for a validated image. Pre-determined values or ranges of values may be stored locally at the imaging apparatus 112, a memory or online in a database.

In another exemplar performance of an aspect of the method 200 relating to a parameter acceptability check 400, a modified bandwidth is identified as a critical parameter and checked against a predetermined value. In one example, the predetermined value is 224 Hz, which is a frequency conventionally used for a field strength of 1.5 T. In other words, the parameter acceptability check 400 checks that a modified bandwidth parameter is greater than or equal to 224Hz. This provides the advantage of ensuring that the imaging protocol with modified protocol parameters will reduce or prevent image distortions.

In some examples, the predetermined values may be Boolean, i.e. indicating whether a parameter is 'true' or 'false', or 'selected' or 'deselected'. For example, checking 404 the critical parameter 3D geometrical distortion correction means against a predetermined value may comprise checking that the 3D geometrical distortion correction means are implemented, i.e. that the parameter is 'true' or 'selected'. This provides the advantage of ensuring that the imaging protocol with modified protocol parameters will reduce and correct distortions.

Checking 404 the field of view against a predetermined value allows the parameter acceptability check 400 to ensure that the field of view covers the subject, or portion of the subject, to be imaged. This prevents wrapping or aliasing when acquiring the image with the modified protocol parameters.

In a further exemplar performance of an aspect of the method 200 relating to a parameter acceptability check 400, a modified sound pressure level is identified as a critical parameter and compared to a predetermined value which corresponds to an upper limit. The upper limit is 110dB(A). This improves the patient experience, in particular by ensuring that the imaging protocol with modified protocol parameters will not cause uncomfortable or disturbing noise levels as perceived by the patient.

In yet further exemplar performances of the method 200, a SAR level is identified 402 as a critical parameter and checked 404 against a predetermined value. This improves the patient experience, in particular by ensuring that the imaging protocol with a modified SAR parameter will not generate uncomfortable or unpleasant heat levels in or around the patient.

The parameter acceptability check according to the present disclosure may act to further mitigate image artefacts, maintain and provide high image quality and optimal performance of an imaging system, and ensure correct adaptation or recalculation of treatment plans based on acquired images. Furthermore, checking modified parameters such as sound pressure level and SAR can ensure a pleasant experience for a patient during image acquisition, reducing anxiety and discomfort. A parameter acceptability check according to the present disclosure may also be applied to other image quality parameters, imaging apparatus functioning parameters and/or radiotherapy system functioning parameters known to the skilled person.

Additional advantages provided by aspects of the method 200 include the ability to modify imaging protocol parameters whilst not placing additional mental or administrative load on the user of the imaging protocol. The user may be a clinician, optionally using the imaging protocol as part of a radiotherapy treatment plan and/or clinical workflow. By enabling modification, customisation and optimisation of imaging protocol parameters, imaging protocols can be modified, customised and optimised specific to each patient and/or each workflow by modifying only the parameters which require modification, whilst providing other necessary parameters or settings as part of the imaging protocol. This means that patient- and/or workflow-specific protocols can be created without having to start from scratch each time. Additionally, this minimises the risk of user error or omission when modifying the parameters.

Any example of the method 200 may also or alternatively be implemented in workflows or with devices configured for functional imaging techniques. For example, for diffusion methods, using the image acceptability checks and parameter acceptability checks disclosed herein, modified protocol parameters such as b-value can be verified to be within a certain range, in order to mitigate any adverse effects to image quality. The method may also be implemented for use in relation to motion-management images. The method may also be implemented to check parameters of functional maps and to compare those to a "validated" map from a validated protocol. In such cases, an alternative/additional method may be used, comprising analysing the output of the images by determining whether the apparent diffusion coefficient (ADC) values are (sufficiently) similar and/or whether the motion estimates are (sufficiently) similar, using the image acceptability check and parameter acceptability check approaches used herein, in order to determine whether the modified protocol parameters are acceptable.

In some examples, in addition to or in place of steps described herein, the method may comprise manual viewing, by a clinician, of a registered image and an acquired image to determine a similarity between the acquired and validated images. In other words, an acquired image acceptability check may comprise manual review of an acquired image by a clinician. For example, the clinician may determine whether the images can be overlaid, and/or determine whether the degree of overlay is sufficient. This may be described as a manual assessment or determination of a similarity metric value or geometrical fidelity between the validated image and the acquired image. The clinician may also perform a visual detection of artefacts in the validated and/or acquired image.

According to an aspect of the present disclosure, the method 200 according to any example described herein may further comprise performing artefact detection in an artefact detection step 210, on at least one of an acquired image and a validated image. The artefact detection step 210 is performed to detect, locate and/or segment artefacts in at least one of the acquired image and the validated image, and thus comprises detecting, locating and/or segmenting artefacts in at least one of the acquired image and the validated image. Additionally or alternatively, the artefact detection step 210 reports an image quality of at least one of the acquired image and the validated image. The reported image quality may be based at least in part on the detection, location and/or segmentation of artefacts in the acquired and/or validated image. The reported image quality may be used as part of the adapting or recalculating of a treatment plan, or as part of subsequent imaging workflows. The image quality may be reported on a qualitative scale, for example as a percentage. The scale may range from 0-100%, preferably from 10-100%. The scale may comprise a minimum or threshold image quality, which the reported image quality must exceed in order for the image to be deemed acceptable, for example within the range of 50-100%, or preferably within 70-90%, or more preferably within 75-85%, or even more preferably a minimum threshold image quality of 80%. For each range, the end points are included, so that for example the range of 50-100% includes the values of 50% and 100%.

In some examples, the artefact detection step 210 is performed by a computer vision tool. The computer vision tool may be based on deep convolutional neural networks and/or conventional image processing algorithms, and may be trained to detect, locate and/or segment artefacts present in images. In other words, the computer vision tool performs a method of artefact detection, location and/or segmentation for images. The computer vision tool is programmed to, and used to, detect, locate and/or segment artefacts in at least one of the acquired image and the validated image, and may also report a probability of an artefact being present in an image. Additionally or alternatively, the computer vision tool may report an image quality which may be based at least in part on the detected, located and/or segmented artefacts. The image quality may be reported on a qualitative scale, for example as a percentage. The computer vision tool may be arranged to detect, locate, and/or segment artefacts based on comparing at least one of the acquired image and the validated image to a template or reference image of an artefact in order to assess whether the artefact associated with the template or reference image is present in the at least one of the acquired image and the validated image.

In one aspect, the computer vision tool suggests or outputs alternative modified protocol parameters, for example based at least in part on a reported image quality of at least one of an acquired image and a validated image. In particular, if a reported image quality for an acquired image is below a threshold quality, for example 80%, the computer vision tool prompts the user to reacquire the image with different parameters, a different sequence choice and/or different actions from a clinician in order to reduce or mitigate detected artefacts. The computer vision tool, therefore, acts as an additional quality assurance measure. In examples where the computer vision tool is unable to suggest different parameters, sequence choices or clinician actions to sufficiently reduce or mitigate artefacts, or in addition to such suggestions, the computer vision tool may provide or suggest alternative clinical approaches to obviate any detrimental effects of the artefact on an imaging workflow. This may be particularly effective for sources of artefacts including but not limited to bulk motion of the patient, respiratory motion, metallic implants and/or excessive radial streaking. For example, to minimise bulk motion of a patient, the computer vision tool may instruct a clinician to calm the patient and ask them to try to remain still. In some examples, to minimise or regulate respiratory motion of a patient, the computer vision tool may instruct a clinician to re-attempt image acquisition whilst implementing respiratory coaching. The clinician may be, for example, a radiographer. In some examples, to minimise artefacts caused by metallic implants, the computer vision tool may suggest utilisation of spin-echo instead of gradient echo images, thereby reducing signal drop-out. In some examples, to minimise or prevent excessive radial streaking, the computer vision tool may suggest acquiring 3D VANE with more spokes.

The computer vision tool may provide various advantages, in addition to those provided by examples of the method described herein. The tool performs artefact detection, provides automated image quality checks, effectively reduces image artefacts or provides workflow-specific guidance on how to reduce image artefacts, reduces the risk of incorrect adaptation or recalculation of treatment plans as a result of image artefacts and may improve the patient experience. The computer vision tool reduces the workload of a clinician in that they are not required to manually review images for artefacts, or determine alternative protocols to reduce artefacts.

The computer vision tool may be used in the determining 204 of whether the modified protocol parameters are acceptable in aspects of the method 200, in one or both of an acquired image acceptability check 300 or parameter acceptability check 400 according to any example described herein. The computer vision tool may also be used in any other steps of the method 200 described herein. Alternative protocol parameters suggested or output by the computer vision tool according to any example described herein may be based at least in part on at least one of an acquired image acceptability check 300 and a parameter acceptability check 400.

A method for medical image acquisition, optionally as part of a clinical workflow, may comprise the method 200 for modifying medical imaging protocol parameters according to any embodiment described herein. The method for image acquisition may further comprise, where the modified protocol parameters are determined to be acceptable, acquiring a subsequent image using the protocol with the acceptable modified protocol parameters. In examples in which the medical imaging is MR imaging, the method comprises acquiring an MR image using the protocol with the acceptable modified protocol parameters. In other examples, such as examples in which the medical imaging is CT imaging, the method comprises acquiring a CT image using the protocol with the acceptable modified protocol parameters. Where the modified protocol parameters are determined to be unacceptable, the method for image acquisition may further comprise one or more of the following steps: receiving alternative modified protocol parameters for the imaging protocol, for example alternative modified protocol parameters output by or from a computer vision tool; acquiring a subsequent image of the subject using the protocol with the alternative modified parameters; and determining a whether the alternative modified protocol parameters are acceptable based on at least one of an acquired image acceptability check and a parameter acceptability check. The computer vision tool may also be used according to any example in the method for medical image acquisition. For example, the computer vision tool may be used to detect, locate and/or segment artefacts in an artefact detection step in the subsequent image acquired using the alternative modified protocol parameters. The computer vision tool may report an image quality of the subsequent acquired image and/or suggest or output further alternative modified protocol parameters or clinician directions based at least in part on a reported quality of the subsequent acquired image and/or a validated image. The method may be repeated, outputting or suggesting further alternative modified parameters until a determination of acceptable modified protocol parameters is reached. A clinical workflow may comprise the method 200 for modifying medical imaging protocol parameters and/or the method for medical image acquisition as described herein.

Figure 5 illustrates a block diagram of one implementation of a radiotherapy system 500. The radiotherapy system 500 comprises a computing system 510 within which a set of instructions, for causing the computing system 510 to perform any one or more of the methods discussed herein, may be executed.

The computing system 510 shall be taken to include any number or collection of machines, e.g. computing device(s), that individually or jointly execute a set (or multiple sets) of instructions to perform any one or more of the methods discussed herein. That is, hardware and/or software may be provided in a single computing device, or distributed across a plurality of computing devices in the computing system. In some implementations, one or more elements of the computing system may be connected (e.g., networked) to other machines, for example in a Local Area Network (LAN), an intranet, an extranet, or the Internet. One or more elements of the computing system may operate in the capacity of a server or a client machine in a client-server network environment, or as a peer machine in a peer-to-peer (or distributed) network environment. One or more elements of the computing system may be a personal computer (PC), a tablet computer, a set-top box (STB), a Personal Digital Assistant (PDA), a cellular telephone, a web appliance, a server, a network router, switch or bridge, or any machine capable of executing a set of instructions (sequential or otherwise) that specify actions to be taken by that machine.

The computing system 510 includes controller circuitry 511 and a memory 513 (e.g., read-only memory (ROM), flash memory, dynamic random access memory (DRAM) such as synchronous DRAM (SDRAM) or Rambus DRAM (RDRAM), etc.). The memory 513 may comprise a static memory (e.g., flash memory, static random access memory (SRAM), etc.), and/or a secondary memory (e.g., a data storage device), which communicate with each other via a bus (not shown).

Controller circuitry 511 represents one or more general-purpose processors such as a microprocessor, central processing unit, accelerated processing units, or the like. More particularly, the controller circuitry 511 may comprise a complex instruction set computing (CISC) microprocessor, reduced instruction set computing (RISC) microprocessor, very long instruction word (VLIW) microprocessor, processor implementing other instruction sets, or processors implementing a combination of instruction sets. Controller circuitry 511 may also include one or more special-purpose processing devices such as an application specific integrated circuit (ASIC), a field programmable gate array (FPGA), a digital signal processor (DSP), network processor, or the like. One or more processors of the controller circuitry may have a multicore design. Controller circuitry 511 is configured to execute the processing logic for performing the operations and steps discussed herein.

The computing system 510 may further include a network interface circuitry 518. The computing system 510 may be communicatively coupled to an input device 520 and/or an output device 530, via input/output circuitry 517. In some implementations, the input device 520 and/or the output device 530 may be elements of the computing system 510. The input device 520 may include an alphanumeric input device (e.g., a keyboard or touchscreen), a cursor control device (e.g., a mouse or touchscreen), an audio device such as a microphone, a graphical user interface (GUI) and/or a haptic input device. The output device 530 may include an audio device such as a speaker, a video display unit (e.g., a liquid crystal display (LCD) or a cathode ray tube (CRT)), and/or a haptic output device. In some implementations, the input device 520 and the output device 530 may be provided as a single device, or as separate devices. The input device 520 may be used to input the modified protocol parameters. In other words, the modified protocol parameters may be input at the input device 520 and/or the input device 520 may be configured to receive the modified protocol parameters.

In some implementations, the computing system 510 includes training circuitry 518. The training circuitry 518 is configured to train a method of detecting, locating and/or segmenting artefacts in MR images. For example, the training circuitry 518 may train a model for performing a method of artefact detection, location and/or segmentation for MR images. Training circuity 518 may also train a model for suggesting imaging protocol parameters, sequence choices and/or clinician actions based at least in part on detected image artefacts. The model may comprise a deep neural network (DNN), such as a convolutional neural network (CNN) and/or recurrent neural network (RNN). Training circuitry 518 may be configured to execute instructions to train a model that can be used to detect, locate and/or segment image artefacts in MR. Training circuitry 518 may also be configured to execute instructions to train a model that can be used to suggest imaging protocol parameters, sequence choices and/or clinician actions based at least in part on detected image artefacts. Training methods for training such models will be known to the skilled person. Training circuitry 518 may be configured to access training data and/or testing data from memory 513 or from a remote data source, for example via network interface circuitry 515. In some examples, training data and/or testing data may be obtained from an external component, such as an image acquisition device 540 and/or treatment device 550. In some implementations, training circuitry 518 may be used to update, verify and/or maintain the model for detecting, locating and/or segmenting image artefacts in MR images. In some implementations, training circuitry 518 may also be used to update, verify and/or maintain the model for suggesting imaging protocol parameters, sequence choices and/or clinician actions based at least in part on detected image artefacts.

In some implementations, the computing system 510 may comprise image processing circuitry 519. Image processing circuitry 519 may be configured to process image data 580 (e.g. images, or imaging data), such as medical images obtained from one or more imaging data sources, a treatment device 550 and/or an image acquisition device 540. Image processing circuitry 519 may be configured to process, or pre-process, image data. For example, image processing circuitry 519 may convert received image data into a particular format, size, resolution or the like. In some implementations, image processing circuitry 519 may be combined with controller circuitry 511. Image processing circuitry 519 may be configured to process image data according to any example of the method 200 described herein. For example, in some examples, image processing circuitry 519 may be configured for an acquired image acceptability check, a structural similarity index metric technique and/or normalised cross correlation. Image processing circuitry 519 may be configured to determine whether the modified protocol parameters are acceptable based on at least one of an acquired image acceptability check and a parameter acceptability check.

In some implementations, the radiotherapy system 500 may further comprise an image acquisition device 540 and/or a treatment device 550. The image acquisition device 540 and the treatment device 550 may be provided as a single device. In some implementations, treatment device 550 is configured to perform imaging, for example in addition to providing treatment and/or during treatment. The treatment device 550 comprises the main radiation delivery components of the radiotherapy system, such as the linac.

The image acquisition device 540 may be configured to perform magnetic resonance imaging (MRI), and may also or alternatively be configured to perform positron emission tomography (PET), or computed tomography (CT). In those examples in which image acquisition device 540 is configured to perform positron emission tomography, the approaches disclosed herein may be applied to PET imaging and images. In those examples in which image acquisition device 540 is configured to perform CT imaging, the approaches disclosed herein may be applied to CT imaging and images. In other examples, the image acquisition device 540 is configured to perform one or more of portal imaging, cone-beam CT, and/or SPECT imaging, and the approaches disclosed herein may be applied to the respective imaging modality.

The image acquisition device 540 may be configured to output image data 580, which may be accessed by the computing system 510. The treatment device 550 may be configured to output treatment data 560, which may be accessed by the computing system 510.

The computing system 510 may be configured to access or obtain treatment data 560, planning data 570 and/or image data 580. Treatment data 560 may be obtained from an internal data source (e.g. from memory 513) or from an external data source, such as the treatment device 550 or an external database. Planning data 570 may be obtained from memory 513 and/or from an external source, such as a planning database. Planning data 570 may comprise information obtained from one or more of the image acquisition device 540 and the treatment device 550. At least one of image data, treatment data and/or planning data may be used by the computer system 510 to adapt or recalculate a treatment plan. The input device 520 may also be configured to receive and/or used to input treatment data 560 and/or planning data 570.

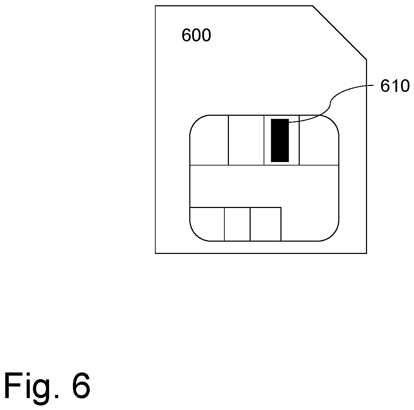

The various methods described above may be implemented by a computer program. The computer program may include computer code (e.g. instructions) 610 arranged to instruct a computer to perform the functions of one or more of the various methods described above. The steps of the methods described above may be performed in any suitable order. For example, artefact detection step 210 of method 200 may be performed before, after, simultaneously or substantially simultaneously with step 220. An acquired image acceptability check according to step 300 may be performed before, after, simultaneously or substantially simultaneously with a parameter acceptability check according to step 400. The computer program and/or the code 610 for performing such methods may be provided to an apparatus, such as a computer, on one or more computer readable media or, more generally, a computer program product 600, depicted in Figure 6. The computer readable media may be transitory or non-transitory. The one or more computer readable media 600 could be, for example, an electronic, magnetic, optical, electromagnetic, infrared, or semiconductor system, or a propagation medium for data transmission, for example for downloading the code over the Internet. Alternatively, the one or more computer readable media could take the form of one or more physical computer readable media such as semiconductor or solid state memory, magnetic tape, a removable computer diskette, a random access memory (RAM), a read-only memory (ROM), a rigid magnetic disc, and an optical disk, such as a CD-ROM, CD-R/W or DVD. The instructions 610 may also reside, completely or at least partially, within the memory 513 and/or within the controller circuitry 511 during execution thereof by the computing system 510, the memory 513 and the controller circuitry 511 also constituting computer-readable storage media.

In an implementation, the modules, components and other features described herein can be implemented as discrete components or integrated in the functionality of hardware components such as ASICS, FPGAs, DSPs or similar devices.

A "hardware component" is a tangible (e.g., non-transitory) physical component (e.g., a set of one or more processors) capable of performing certain operations and may be configured or arranged in a certain physical manner. A hardware component may include dedicated circuitry or logic that is permanently configured to perform certain operations. A hardware component may comprise a special-purpose processor, such as an FPGA or an ASIC. A hardware component may also include programmable logic or circuitry that is temporarily configured by software to perform certain operations.

In addition, the modules and components can be implemented as firmware or functional circuitry within hardware devices. Further, the modules and components can be implemented in any combination of hardware devices and software components, or only in software (e.g., code stored or otherwise embodied in a machine-readable medium or in a transmission medium).

Unless specifically stated otherwise, as apparent from the following discussion, it is appreciated that throughout the description, discussions utilizing terms such as " receiving", "determining", "checking", "registering", "assessing ", "suggesting", "validating", "evaluating", "outputting", "storing", "obtaining", "providing" or the like, refer to the actions and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

It is to be understood that the above description is intended to be illustrative, and not restrictive. Many other implementations will be apparent to those of skill in the art upon reading and understanding the above description. Although the present disclosure has been described with reference to specific example implementations, it will be recognized that the disclosure is not limited to the implementations described, but can be practiced with modification and alteration within the spirit and scope of the appended claims. In particular, features and steps described herein may be combined in any arrangement, and in any order. Accordingly, the specification and drawings are to be regarded in an illustrative sense rather than a restrictive sense. The scope of the disclosure should, therefore, be determined with reference to the appended claims, along with the full scope of equivalents to which such claims are entitled.

Examples of the present disclosure are set out in the following numbered clauses:
1. A computer implemented method for modifying medical imaging protocol parameters, comprising:
   receiving modified protocol parameters for an imaging protocol; and
   determining whether the modified protocol parameters are acceptable based on at least one of an acquired image acceptability check and a parameter acceptability check.
2. The method of clause 1, further comprising updating protocol parameters based on the modified protocol parameters.
3. The method of any preceding clause, further comprising outputting and/or storing the determination of whether the modified protocol parameters are acceptable.
4. The method of any preceding clause, wherein the acquired image acceptability check comprises:
   acquiring an image of a subject using the protocol with the modified protocol parameters;
   determining a similarity metric value between the acquired image and a validated image; and
   determining whether the modified protocol parameters are acceptable based at least in part on the similarity metric value.
5. The method of clause 4, wherein the similarity metric value is associated with a geometrical transformation between the acquired image and the validated image.
6. The method of clause 5, wherein the geometrical transformation comprises a deformation vector field.
7. The method of clause 6, wherein determining the geometrical transformation comprises determining at least one of an x, y and/or z displacement component of the deformation vector field and/or a scalar length of at least one of the displacement components.
8. The method of clause 6 or 7, wherein the determining whether the modified protocol parameters are acceptable is based at least in part on at least one of an x, y and/or z displacement component of the deformation vector field and/or a scalar length of at least one of the displacement components.
9. The method of any of clauses 6 to 8, wherein the modified protocol parameters are determined to be acceptable if a scalar length of a threshold proportion of the x, y and/or z displacement components are less than or equal to a maximum scalar length.
10. The method of clause 9, wherein the threshold proportion lies within 60% to 100%, preferably wherein the threshold proportion lies within 80% to 100%, more preferably wherein the threshold proportion lies within 90% to 100%, even more preferably wherein the threshold proportion is about 95%.
11. The method of any of clauses 9 to 10, wherein the maximum scalar length lies within 0.5mm to 4mm, preferably wherein the maximum scalar length lies within 1.5mm to 2.5mm, more preferably wherein the maximum scalar length is about 2mm.
12. The method of any of clauses 9 to 11, wherein the maximum scalar length is based on a resolution of the validated image.
13. The method of any of clauses 5 to 12, wherein determining a geometrical transformation between the acquired image and a validated image comprises attempting to register the acquired image to the validated image.
14. The method of clause 13, wherein if an attempt to register the acquired image to the validated image is unsuccessful and/or a geometrical transformation cannot be determined, the modified protocol parameters are determined to be unacceptable.
15. The method of any of clauses 5 to 14, wherein the validated image is acquired using the imaging protocol with unmodified or validated protocol parameters.
16. The method of any of clauses 4 to 15, wherein the similarity metric value is associated with a structural similarity index metric or normalized cross correlation metric between the acquired image and the validated image.
17. The method of any preceding clause in which the parameter acceptability check comprises identifying if a modified protocol parameter is a critical parameter or performance parameter and checking an identified modified protocol parameter against a predetermined value.
18. The method of clause 17 wherein identifying if a modified protocol parameter is a critical parameter comprises identifying if the modified protocol parameter corresponds to at least one of: bandwidth, 3D geometrical distortion, field of view, sound pressure level and/or specific absorption rate.
19. The method of clause 17 or clause 18, wherein identifying if a modified protocol parameter is a performance parameter comprises identifying if the modified protocol parameter corresponds to at least one of: a signal-to-noise ratio, contrast, view of the body, time of the acquisition, resolution of the acquisition, and/or fat suppression.
20. The method of any preceding clause, further comprising outputting and/or storing the determination of whether the modified protocol parameters are acceptable, and optionally storing the modified protocol parameters with their determination.
21. The method of any preceding clause, wherein the modified protocol parameters are received from a user input device.
22. The method of any preceding clause, further comprising performing artefact detection on an acquired image.
23. The method of clause 22 wherein performing the artefact detection comprises detecting, locating and/or segmenting artefacts in at least one of the acquired image and the validated image.
24. The method of clause 22 or 23, wherein performing the artefact detection comprises reporting an image quality of at least one of the acquired image and the validated image.
25. The method of clause 24, wherein a computer vision tool outputs an alternative modified protocol parameter based at least in part on a reported image quality of the acquired image and/or the validated image.
26. A method for medical image acquisition, comprising:
   the method for modifying medical imaging protocol parameters of any preceding clause; wherein if the modified protocol parameters are determined to be acceptable, acquiring a subsequent image using the protocol with the acceptable modified protocol parameters.
27. The method of any preceding clause, wherein the medical imaging is magnetic resonance imaging.
28. A computer readable medium comprising computer-executable instructions which, when executed by a processor, cause the processor to perform the method of any of clauses 1 to 27.
29. A system for medical imaging, comprising:
   a medical imaging apparatus;
   a processor; and
   a computer readable medium comprising computer-executable instructions which, when executed by the processor, cause the processor to perform the method of any of clauses 1 to 27.
30. A radiotherapy device comprising the system for medical imaging of clause 29.

## Claims

1. A computer implemented method for modifying medical imaging protocol parameters, comprising:
receiving modified protocol parameters for an imaging protocol; and
determining whether the modified protocol parameters are acceptable based on at least one of an acquired image acceptability check and a parameter acceptability check.

2. The method of claim 1, further comprising updating protocol parameters based on the modified protocol parameters.

3. The method of any preceding claim, wherein the acquired image acceptability check comprises:
acquiring an image of a subject using the protocol with the modified protocol parameters;
determining a similarity metric value between the acquired image and a validated image; and
determining whether the modified protocol parameters are acceptable based at least in part on the similarity metric value.

4. The method of claim 3, wherein the similarity metric value is associated with a geometrical transformation between the acquired image and the validated image.

5. The method of claim 4, wherein the geometrical transformation comprises a deformation vector field.

6. The method of claim 5, wherein determining the geometrical transformation comprises determining at least one of an x, y and/or z displacement component of the deformation vector field and/or a scalar length of at least one of the displacement components.

7. The method of claim 5 or 6, wherein the determining whether the modified protocol parameters are acceptable is based at least in part on at least one of an x, y and/or z displacement component of the deformation vector field and/or a scalar length of at least one of the displacement components.

8. The method of any of claims 5 to 7, wherein the modified protocol parameters are determined to be acceptable if a scalar length of a threshold proportion of the x, y and/or z displacement components are less than or equal to a maximum scalar length, optionally wherein at least one of:
A. the threshold proportion lies within 60% to 100%, preferably wherein the threshold proportion lies within 80% to 100%, more preferably wherein the threshold proportion lies within 90% to 100%, even more preferably wherein the threshold proportion is about 95%;
B. the maximum scalar length lies within 0.5mm to 4mm, preferably wherein the maximum scalar length lies within 1.5mm to 2.5mm, more preferably wherein the maximum scalar length is about 2mm; and/or
C. the maximum scalar length is based on a resolution of the validated image.

9. The method of any of claims 4 to 8, wherein at least one of:
A. determining a geometrical transformation between the acquired image and a validated image comprises attempting to register the acquired image to the validated image, optionally wherein if an attempt to register the acquired image to the validated image is unsuccessful and/or a geometrical transformation cannot be determined, the modified protocol parameters are determined to be unacceptable; and/or
B. the validated image is acquired using the imaging protocol with unmodified or validated protocol parameters.

10. The method of any of claims 3 to 9, wherein the similarity metric value is associated with a structural similarity index metric or normalized cross correlation metric between the acquired image and the validated image.

11. The method of any preceding claim in which the parameter acceptability check comprises identifying if a modified protocol parameter is a critical parameter or performance parameter and checking an identified modified protocol parameter against a predetermined value, optionally wherein at least one of:
identifying if a modified protocol parameter is a critical parameter comprises identifying if the modified protocol parameter corresponds to at least one of: bandwidth, 3D geometrical distortion, field of view, sound pressure level and/or specific absorption rate; and/or
identifying if a modified protocol parameter is a performance parameter comprises identifying if the modified protocol parameter corresponds to at least one of: a signal-to-noise ratio, contrast, view of the body, time of the acquisition, resolution of the acquisition, and/or fat suppression.

12. The method of any preceding claim, further comprising performing artefact detection on an acquired image, optionally wherein at least one of:
performing the artefact detection comprises detecting, locating and/or segmenting artefacts in at least one of the acquired image and the validated image; and/or
performing the artefact detection comprises reporting an image quality of at least one of the acquired image and the validated image, further optionally wherein a computer vision tool outputs an alternative modified protocol parameter based at least in part on a reported image quality of the acquired image and/or the validated image.

13. A method for medical image acquisition, comprising:
the method for modifying medical imaging protocol parameters of any preceding claim; wherein if the modified protocol parameters are determined to be acceptable, acquiring a subsequent image using the protocol with the acceptable modified protocol parameters.

14. A computer readable medium comprising computer-executable instructions which, when executed by a processor, cause the processor to perform the method of any of claims 1 to 13.

15. A system for medical imaging, comprising:
a medical imaging apparatus;
a processor; and
a computer readable medium comprising computer-executable instructions which, when executed by the processor, cause the processor to perform the method of any of claims 1 to 13.
